# EUROPÄISCHE PATENTSCHRIFT

(11) **EP 2 229 236 B1**
(45) Veröffentlichungstag und Bekanntmachung des Hinweises auf die Patenterteilung: **15.02.2012**
(21) Anmeldenummer: 08861624.8
(22) Anmeldetag: 16.12.2008
(51) Int. Cl.: C08J 7/04, G01N 33/543, B01L 3/00, C08F 8/00, C08J 7/12

(54) **VERFAHREN ZUR FUNKTIONALISIERUNG VON MULTI-WELL-PLATTEN FÜR BIOCHEMISCHE ANALYSEN UND ZELLKULTUREXPERIMENTE**
PROCESS FOR FUNCTIONALISING MULTIWELL PLATE FOR BIOCHEMICAL ANALYSES AND CELL CULTURE EXPERIMENTS
PROCEDÉ POUR PRODUIRE DES PLAQUETTEs À PUITS MULTIPLES POUR ANALYSES BIOCHIMIQUES ET ESSAIS DE CULTURE CELLULAIRE

(30) Priorität: 19.12.2007 DE 102007055865
(43) Veröffentlichungstag der Anmeldung: 22.09.2010
(73) Patentinhaber: Leibniz-Institut für Polymerforschung Dresden e.V., 01069 Dresden (DE)
(72) Erfinder: POMPE, Tilo, 01217 Dresden (DE); LEHMANN, Kristina, 13189 Berlin (DE); Dr. rer. nat. Mirko Nitschke, 01217 Dresden (DE); Prof. Dr. rer. nat. Carsten Werner, 01069 Dresden (DE)
(74) Vertreter: Sperling, Thomas
(86) Internationale Anmeldenummer: PCT/EP2008/067647
(87) Internationale Veröffentlichungsnummer: WO 2009/077535

(56) Entgegenhaltungen:
- EP-A- 0 930 331
- WO-A-2004/065009
- DE-A1- 10 321 042
- US-A1- 2007 154 348
- US-A1- 2007 264 155

## Beschreibung

Die Erfindung betrifft ein Verfahren zur Funktionalisierung von Multi-Well-Platten aus Polystyren oder aus Polypropylen für biochemische Analysen und Zellkulturexperimnente nach Anspruch 1.

Als Trägermaterial für biochemische Analysen und Zellkulturexperimente kommen insbesondere 96-Well-Platten aus Polystyren in zahlreichen Anwendungen zum Einsatz. Eine Vielzahl von Analysegeräten sind deshalb auf deren standardisierte Größe abgestimmt. Die Oberflächeneigenschaften dieser und ähnlicher kommerziell verfügbaren Träger werden beispielsweise durch Niederdruckplasma-Verfahren modifiziert, um bessere Eigenschaften in den entsprechenden Experimenten zu erreichen. Damit besitzen die herkömmlichen Platten allerdings sehr undefinierte physikochemische Oberflächeneigenschaften, welche in den nachfolgenden biochemischen und zellbiologischen Experimenten zu ungeklärten und damit auch unkontrollierten Wechselwirkungen von Biomolekülen mit den Substraten führen. Aus einigen Untersuchungen auf dem Gebiet der Biomaterialwissenschaft, wie zum Beispiel bei McClary et al. Modulation fibroblast adhesion, spreading, and proliferation using self-assembled monolayer films of alkylthiolates on gold. J. Biomed. Mater. Res 2000; 50: 428 - 439**,** beschrieben, ist bekannt, dass die Bindungschemie und die Art der Wechselwirkungen, die von der Substratoberfläche aus wirken, die Wirkung der angelagerten Biomoleküle sehr empfindlich beeinflussen können. Dabei kann es in vielen Fällen zur Änderung der Konformation, der Orientierung, der Konzentration, und der Mobilität der Biomoleküle an der Oberfläche kommen, wie es auch durch Keselowsky et al. in Integrin binding specificity regulates biomaterial surface chemistry effects on cell differentiation, Proc. Natl. Acad. Sci. USA 2005; 102:5953-5957**,** offenbart ist. Wie darüber hinaus, insbesondere von Green et al. in der Publikation Competitive protein adsorption as observed by surface plasmon resonance, Biomaterials 1999; 20:385 bis 391, beschrieben wurde, kann sich im Laufe der Experimente in unterschiedlicher und undefinierter Weise auch die Zusammensetzung der adsorbierten Biomolekülschicht an der Oberfläche ändern. Dieses Problem wird bisher bei vielen Experimenten vernachlässigt, was zu fehlerhaften Ergebnissen und Interpretationen führen kann.

Wie bereits erläutert, sind diese Probleme und Erscheinungen gut bekannt und in Untersuchungen an gut charakterisierten Modelloberflächen dokumentiert. Die fehlende Berücksichtigung dieser bekannten Probleme in der Laborpraxis bei biochemischen Standardanalysen und Zellkulturexperimenten dürfte auf zweierlei Gründe zurückzuführen sein. Einerseits sind sich viele Forscher der Problematik nicht in ausreichendem Maße bewusst, weil sie nur über relativ wenige Kenntnisse auf dem Gebiet der Werkstoffkunde verfügen. Gleichzeitig ist auch nur eine begrenzte Zahl an Werkstoffen erhältlich, welche unterschiedliche Anlagerungsmechanismen von Biomolekülen an Multi-Well-Platten, wie zum Beispiel 96-Well-Platten, erlauben.

Um Biomoleküle gezielt an den Oberflächen der Multi-Well-Platten zu koppeln, sind chemische Modifikationen notwendig. Dazu sind aus dem Stand der Technik allerdings bisher nur wenige Verfahren bekannt.

Eine zweite nachteilige Einschränkung herkömmlicher Zellkulturforschung besteht darin, dass mikrostrukturierte Funktionalisierungen in Hochleistungs-Zellkulturexperimenten für Standardzelluntersuchungen, insbesondere auf PS-96-Well-Platten, fast nicht verbreitet sind.

Eine Reihe von interessanten Besonderheiten des Zellverhaltens bei lateraler Behinderung ist aus zahlreichen Untersuchungen an Modelloberflächen bekannt. Dabei wurde von Lehnert et al. in Cell behaviour on micropatterned substrata: limits of extracellular matrix geometry for spreading and adhesion, J. Cell. Sci. 2003; 117:41-52 beziehungsweise von Tan et al. in Effects of Channel Size, Cell Type and Matrix Composition on Pattern Integrity, Tissue Eng 2003; 9:255-267 nicht nur für die Zelladhäsion und die Zellmorphologie eine Abhängigkeit von der Größe der Mikrostrukturen gezeigt.

Auch die Zellfunktion und Zelldifferenzierung könnte empfindlich durch die Mikrostrukturen beeinflusst werden. Bekannte Beispiele dafür sind das Umschalten zwischen Apoptose, Zellteilung und kapillarartiger Rohrbildung von Endothelzellen, welches bei Chen et al. in der Druckschrift Geometrical Control of Cell Life and Death, Science 1997; 276: 1425 - 1428**,** beschrieben ist.

In der WO 2007/078873 A1 wird ein Verfahren zur Bereitstellung von Multi-Well-Platten für biochemische Analysen und Zellkulturexperimente offenbart. Die Multi-Well-Platten werden mittels eines Maleinsäure-Copolymers funktionalisiert, dass als Lösung aus einem wasserfreien und apriotischen Lösungsmittel auf die zunächst mit Aminogruppen funktionalisierten Multi-Well-Platten aufgebracht und getrocknet wird. Die Aminogruppen des Substrats werden durch Silanisierung eingeführt. Während des Verfahrens werden die Multi-Well-Platten zur Wiederherstellung der Anhydrid-Gruppen am Malein-Copolymer wärmebehandelt. Die Wärmebehandlung findet nach einem teilweisen Blocken der reaktiven Anhydrid-Einheiten und vor dem Einwirken des Polymers auf das Substrat statt.

In der DE 103 15 930 A1 wird ein Verfahren zum Funktionalisieren von künstlichen Zellkulturträgern mit Maleinsäure-Anhydrid-Copolymer beschrieben, bei dem die Erzeugung der Aminogruppen auf dem Substrat durch einen Plasmaprozess in Ammoniakgas erfolgt. Das Maleinsäure-Copolymer wird aus einem apriotischen, wasserfreien Lösungsmittel aufgebracht und der funktionalisierte Träger ohne abschließende Wärmebehandlung verwendet.

In der Druckschrift FTIR spectroscopic studies of interfacial reactions between amino functionalized silicon surfaces and molten maleic anhydride copolymers, Macromol. Chem. Phys. 1999, 200: 852 - 857**,** ist von **Bayer et. al** eine IR-spektroskopische Untersuchung der kovalenten Anbindung von Maleinsäure-Copolymeren an aminofunktionalsierten Substraten offenbart. Dabei werden durch mehrstündiges Heizen im Vakuum die Anhydridfunktionen des Copolymers durch Rezyklisieren zurückgebildet. Das Heizen stellt keinen abschließenden Verfahrensschritt bei der Herstellung einer funktionalisierten Multi-Well-Platte dar.

Auch in den Druckschriften DE 103 21 042 A, US 2006/0257919 A1 und DE 100 48 822 A1 werden Verfahren zur Funktionalisierung von Substraten mittels Maleinsäure-Copolymeren beschrieben.

In der DE 103 21 042 A wird ein zur medizinischen Diagnostik geeignetes Probengefäß mit einer Trägerplatte und Reaktionskammer für Analysen offenbart.

Aus der US 2006/0257919 A1 ist ein Verfahren zur Herstellung eines Substrates zur Anbindung von Molekülen bekannt, wobei das Substrat eine reaktive Oberfläche aufweist, mit der Polymerbeschichtungen mit funktionalen Gruppen kovalent gekoppelt werden. Außerdem dient das Substrat der Bindung verschiedener Biomoleküle an polymerbeschichtete Oberflächen.

In der DE 100 48 822 A1 wird ein Verfahren zum Immobilisieren von Lipidschichten auf Oberflächen, insbesondere pulverförmiger Festkörper, beschrieben. Dabei wird die Festkörperoberfläche mit Molekülen derart modifiziert, dass eine hydrophile Fläche ausgebildet wird. In einem zweiten Verfahrensschritt werden auf der modifizierten Oberfläche Lipidschichten deponiert.

Die meisten im Stand der Technik bekannten, auf Modelloberflächen angewendeten Techniken der Präparation von Mikrostrukturen sind schwer auf 96-Well-Platten zu übertragen oder weisen einen Mangel an Oberflächenmodifikationen auf, die in der biofluiden Umgebung beständig sind. Wie zum Beispiel bei Falconnet et al., Surface engineering approaches to micropattern surfaces for cell-bases assays, Biomaterials 2006; 27:3044-3063**,** zusammengefasst, werden solche Mikrostrukturen durch Techniken gebildet, zu denen die Lithographie, der Mikrokontaktdruck, die Mikrofluid-Technik, die Photoaktivierung, Plasma- oder Laser-anwendende Oberflächenmodifizierungen und auch Drucktechniken, wie Tintenstrahl oder Siebdruck, zählen.

Die Aufgabe der Erfindung besteht darin, eine Multi-Well-Platte für biochemische und Zellkulturanalysen bereitzustellen, bei der die Oberfläche in einer Weise funktionalisiert ist, dass Biomoleküle gezielt an der Oberfläche der Multi-Well-Platte gekoppelt werden können.

Die Aufgabe der Erfindung wird durch ein Verfahren zur Funktionalisierung von Multi-Well-Platten aus Polystyren oder aus Polypropylen für biochemische Analysen und Zellkulturexperimente gelöst, das die folgenden Verfahrenschritte umfasst:
a) Behandlung der Multi-Well-Platte , mittels eines Ammoniak-Niederdruckplasmas, so dass reaktive Aminogruppen an der Plattenoberfläche entstehen;
b) Aufbringen einer wässrigen oder alkoholischen Lösung eines Maleinsäureanhydrid-Copolymers;
c) Eintrocknen der Lösung des Maleinsäureanhydrid-Copolymers auf der Oberfläche;
d) Wärmebehandlung zur kovalenten Bindung des Maleinsäureanhydrid-Copolymers an die Multi-Well-Platte;
e) Spülen mit wässriger Lösung zur Beseitigung von ungebundenen und wasserlöslichem Maleinsäureanhydrid-Copolymer; und
f) Wärmebehandlung zur Wiederherstellung der Reaktivität der Anhydrid-Gruppen am Maleinsäureanhydrid-Copolymer.

Die Konzeption der Erfindung besteht darin, dass eine standardisierte Multi-Well-Platte für biochemische Analysen und Zellkulturexperimente in ihren Oberflächeneigenschaften so verändert wird, dass gewünschte Biomoleküle kovalent oder nicht kovalent angebunden werden können. Über die Wahl des Copolymers für die Beschichtung können die sekundären Wechselwirkungen mit Biomolekülen gezielt beeinflusst werden. Das erfindungsgemäß angewendete Verfahren beinhaltet die Modifizierung mit Maleinsäureanhydrid-Copolymeren, welche eine Kopplungsmöglichkeit zahlreicher Biomoleküle, einschließlich Peptide, Proteine, Polysaccharide und andere bioaktive Moleküle, beinhaltet. Zusätzlich kann mit der gezielten Variation des Comonomers der eingesetzten Copolymere eine unterschiedliche Dichte an kopplungsfähigen Gruppen erreicht sowie die sekundäre Wechselwirkung zwischen Oberfläche und Biomolekülen mittels polarer und hydrophober Wechselwirkungskräfte variiert werden.

Bevorzugt eingesetzte Maleinsäureanhydrid-Copolymere sind zum Beispiel: Poly(styren-alt-maleinsäureanhydrid) PSMA, Poly(propen-alt maleinsäureanhydrid) PPMA oder Poly(ethylen-alt-maleinsäureanhydrid) PEMA. Wichtig ist dabei, dass nach dem Spülschritt e) die Reaktivität der Anhydrid-Gruppen gemäß Schritt f) wieder hergestellt wird. Bei Anwendung von Multi-Well-Platten aus Polystyren erfolgt die Wiederherstellung der Reaktivität der Anhydridgruppen bevorzugt über eine 48-stündige Wärmebehandlung bei einer Temperatur von 90 °C. Kommen dagegen Multi-Well-Platten aus Polypropylen zur Anwendung, kann die Wärmebehandlung vorteilhafterweise bei 120 °C in nur 2 Stunden durchgeführt werden. Vorteilhafterweise können an die Anhydridgruppen der Copolymere Proteine, Peptide und andere bioaktive Moleküle über freie Aminogruppen spontan kovalent gebunden werden. Des Weiteren können an die Anhydridgruppen über freie Hydroxyl-(OH)-Gruppen auch andere bioaktive Moleküle, wie Polysaccharide, kovalent gebunden werden. Die Anbindung mittels der Hydroxylgruppen, wie bei Polysacchariden, an die Anhydridgruppen wird in der Ausführung der Erfindung mit Polystyrenplatten wiederum vorzugsweise durch eine nachfolgende 48-stündige Wärmebehandlung bei 90 °C realisiert, während bei Anwendung von Polypropylenplatten die Wärmebehandlung bevorzugt bei 120 °C und in nur 2 Stunden erfolgt.

Je nach Hydrolysierungszustand der Anhydridgruppe kann die Funktionalisierung der Multi-Well-Platte an der beschichteten Oberfläche außer durch kovalente auch durch nicht kovalente Kopplung von Biomolekülen erfolgen. So können an den hydrolysierten Polymeroberflächen bioaktive Moleküle, wie Proteine oder Polysaccharide, adsorptiv über das Zusammenspiel verschiedener zwischenmolekularer Wechselwirkungen gebunden werden.

In einer weiterführenden Ausgestaltung der Erfindung ist auch eine Strukturierung der Oberflächenfunktionalisierungen vorgesehen. So können in dieser Ausgestaltung der Erfindung auf erfindungsgemäß beschichteten Multi-Well-Platten laterale Strukturen der Oberflächenfunktionalisierungen im Mikrometerbereich erhalten werden. Dazu wird zunächst adsorptiv aus einer wässrigen Lösung eine Dünnschicht von Polyoxyethylene-polyoxypropylene-Block-Copolymeren aufgebracht. Diese wird vorzugsweise in einem Vernetzungsschritt mittels eines Niederdruck-Argonplasmas lokal an die Copolymerschicht kovalent angebunden. Dafür ist die Verwendung einer Maske vorgesehen, die entsprechend vorgefertigte Öffnungen bereithält, durch welche das Niederdruck-Argonplasma hindurchdringen kann. Bevorzugt dienen als Masken Template aus Siliziumwafern. In einem nachfolgenden Spülschritt in Wasser werden nicht gebundene Polyoxyethylene-polyoxypropylene-BlockCopolymere abgelöst. Vorteilhafterweise können auf diese Weise lateral chemisch heterogene Strukturen auf der Oberfläche der Platte in einem Größenbereich von 5 µm bis 500 µm erhalten werden.

Vorteilhaft werden in den darauf folgenden Funktionalisierungsschritten die Flächenbereiche des belassenen Maleinsäureanhydrid-Copolymers im nicht mit Polyoxyethylene-polyoxypropylene-Block-Copolymeren beschichteten Bereich der Platten-Oberfläche zur der bereits erwähnten kovalenten und nicht kovalenten Kopplung von Proteinen, Peptiden, Polysacchariden und anderen bioaktiven Molekülen genutzt. Dieses Prinzip ist zum Beispiel für das Wachstum von Zellen unter lateraler Beschränkung interessant. Aufgrund der proteinresistenten Eigenschaften des Polyoxyethylene-polyoxypropylene-Block-Copolymers kann beispielsweise die nachfolgende Adsorption von Zelladhäsionsproteinen - und somit das Zellwachstum - nur in den Zwischenbereichen mit den funktionalen Maleinsäureanhydrid-Copolymeren erfolgen.

Das erfindungsgemäße Verfahren ist nicht nur für die weit verbreiteten und sehr vielseitig eingesetzten 96-Well Polystyren-Platten einsetzbar, sondern auch für andere Konfigurationsgrößen wie 6-Well, 12-Well, 24-Well und 48-Well. Auch eine Modifizierung von 384-Well-Platten ist möglich.

Alternativ können mit diesem Verfahren außer die zur Zeit weit verbreiteten Polystyren-Platten auch neuerdings zum Einsatz kommende Polypropylen-Platten modifiziert werden, wobei als Multi-Well-Platten Polypropylen-Platten der Konfigurationsgrößen 96-Well, 6-Well, 12-Well, 24-Well,48-Well oder 384-Well eingesetzt werden.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Fig.1:**: ein allgemeines Verfahrensschema zur Funktionalisierung und Mikrostrukturierung von Multi-Well-Platten;
- **Fig. 2:**: eine Gleichung für die Hydrolyse und die Wiedergewinnung der Anhydridfunktionen an Maleinsäureanhydrid-Copolymeren;
- **Fig. 3:**: ein hoch aufgelöstes Kohlenstoff-C₁ₛ-Spektrum einer typischen Probe von einer Polystyren(PS)-Oberfläche, beschichtet mit Poly(ethylen-alt-maleinsäureanhydrid) PEMA nach der Niederdruck-Ammoniakplasmabearbeitung;
- **Fig.4:**: eine Poly(ethylen-*alt*-maleinsäureanhydrid) funktionalisierte Polystyren-Oberfläche nach einer Strukturierung mit Polyoxyethylene-polyoxypropylene-Block-Copolymer.
- **Tabelle 1:**: die Ergebnisse der Röntgenphotoelektronenspektroskopie (XPS)-Quantifizierung von Poly(ethylen-*alt*-maleinsäureanhydrid) (PEMA), Poly(propen-*alt*-maleinsäureanhydrid) (PPMA) auf Aminfunktionalisierten Polystyrenoberflächen.
- **Tabelle 2:**: die Ergebnisse der Ablösung von dicken Poly(ethylen-*alt* maleinsäureanhydrid)- (PEMA), Poly(propen-*alt*-maleinsäureanhydrid)- (PPMA) und Poly(styren-*alt*-maleinsäureanhydrid)- (PSMA)Schichten auf Aminfunktionalisierten Oberflächen von Ellipsometriemessungen der Schichtdicke.
- **Tabelle 3:**: die Ergebnisse der mikroskopischen Helligkeitsmessung von fluoreszentmarkiertem Rinderserumalbumin nach dessen Adsorption auf Poly(ethylen-*alt*-maleinsäureanhydrid) PEMA-Oberflächen mit Polyoxyethylene-polyoxypropylene-Block-Copolymer (PEO) Strukturen.

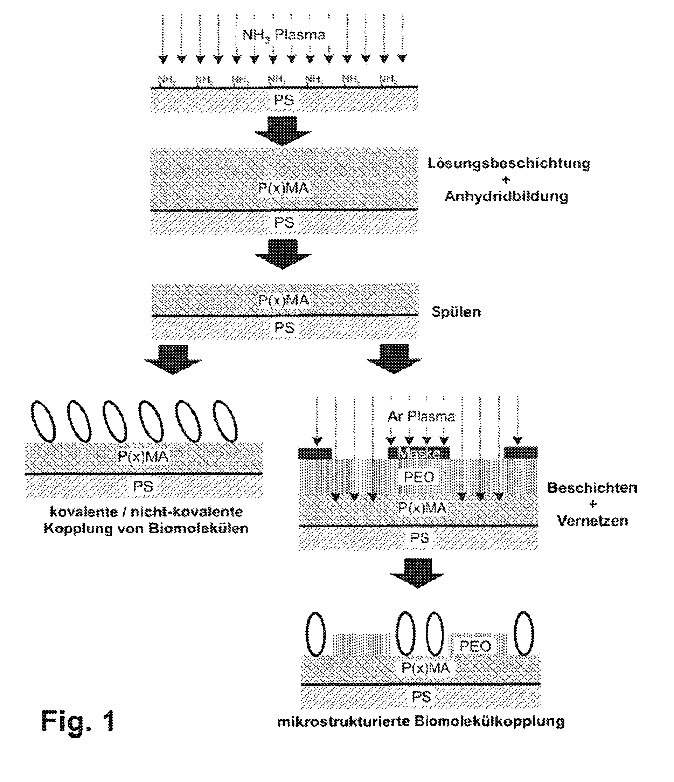

Gemäß dem allgemeinen Verfahrensschema in Fig. 1 wird die Multi-Well-Platte mittels eines Ammoniak-Niederdruckplasmas behandelt, so dass reaktive Aminogruppen an der Plattenoberfläche entstehen. Im nächsten Schritt (Lösungsbeschichtung und Anhydridbildung) wird eine wässrige oder alkoholische Lösung eines Maleinsäureanhydrid-Copolymers mit der allgemeinen Formel P(x)MA auf die behandelte Seite zunächst aufgebracht und die Lösung anschließend eingetrocknet. Durch eine Wärmebehandlung wird schließlich die kovalente Bindung des Maleinsäureanhydrids an die Multi-Well-Platte durch die Regenerierung der Anhydridgruppen hergestellt.

Diesem Schritt folgt das Spülen mit Wasser zur Lösung von ungebundenen und wasserlöslichem Maleinsäureanhydrid-Copolymer und schließlich die Wärmebehandlung zur Wiederherstellung der Reaktivität der Anhydrid-Gruppen am Maleinsäureanhydrid-Copolymer.

Je nach Hydrolysierungszustand der Anhydridgruppe kann die Funktionalisierung der Multi-Well-Platte an der beschichteten Oberfläche durch kovalente oder nicht kovalente Kopplung von Biomolekülen erfolgen, wie es im linken unteren Teil der Fig. 1 skizziert wird.

Andererseits können der Beschichtung der Multi-Well-Platte gemäß dem unteren rechten Abschnitt der Fig. 1 auch Schritte zur Mikrostrukturierung folgen, wobei auf der erfindungsgemäß beschichteten Multi-Well-Platte laterale Strukturen der Oberflächenfunktionalisierungen im Mikrometerbereich erhalten werden. Dazu wird zunächst Polyoxyethylene-polyoxypropylene-Block-Copolymer (PEO) aus einer Lösung adsorptiv aufgebracht und in einem Vernetzungsschritt mittels eines Niederdruck-Argonplasmas lokal angebunden. Dafür ist die Verwendung einer Maske vorgesehen, die entsprechend vorgefertigte Öffnungen bereithält, durch welche das Niederdruck-Argonplasma hindurchdringen kann. In einem nachfolgenden Spülschritt wird ungebundenes Polyoxyethylene-polyoxypropylene-Block-Copolymer entfernt. Anschließend können die lokal belassenen Maleinsäureanhydridcopolymere, wie beschrieben, zur kovalenten und nicht kovalenten Kopplung von Proteinen, Peptiden, Polysacchariden und anderen bioaktiven Molekülen genutzt werden.

Als Multi-Well-Platten wurden Polystyren(PS)-96-Well-Platten (µClear; Greiner Bio-One, Frickenhausen, Deutschland) im Niederdruck-Ammoniakplasma bearbeitet, um freie Aminogruppen auf der Oberfläche der PS 96-Well-Platten herzustellen. Plasmabearbeitungen wurden in einem Computer-gesteuerten MicroSys-Apparat durch Roth & Rau (Wüstenbrand, Deutschland) durchgeführt. Die zylindrische Vakuumkammer, aus reinem Stahl hergestellt, hat einen Durchmesser von 350 mm und eine Höhe von 350 mm. Der Niederdruck, der mit einer Turbomolekularpumpe erreicht wurde, war < 10⁻⁷ mbar. An der Spitze der Kammer wurde eine 2,46 GHz-Elektronen-Zyklotronresonanz(ECR)-Plasmaquelle RR 160 von Roth & Rau mit einem Durchmesser von 160 mm und einer maximalen Leistung von 800 W montiert. Die Plasmaquelle wurde in einem pulsierenden Modus betrieben. Die Prozessgase wurden in das aktive Volumen der Plasmaquelle durch ein Gasflusssteuersystem eingeführt. Wenn die Plasmaquelle angeschaltet war, wurde der Druck durch ein kapazitives Vakuummessgerät gemessen. Die Proben wurden durch ein Load-Lock-System eingeführt und auf einem geerdeten Aluminiumhalter in der Nähe des Zentrums der Kammer platziert. Die Entfernung zwischen den Proben und dem Anregungsvolumen der Plasmaquelle betrug etwa 200 mm.

Bei der Plasmabehandlung betrug die Leistung 400 W, die Pulsfrequenz 1000 Hz, der Tastgrad 5 %, der Ammoniakgasfluss 15 Standard-cm³ pro Minute und der Druck 7 * 10⁻³ mbar. Die Behandlungszeiten wurden im Bereich von 50 s bis 600 s variiert, um optimale Bedingungen zu bestimmen. Auf der Basis des Ergebnisses von entsprechenden Optimierungsversuchen wurde eine Zeit von 300 s als Behandlungszeit für die Niederdruck-Ammoniakplasma-Funktionalisierung gewählt.

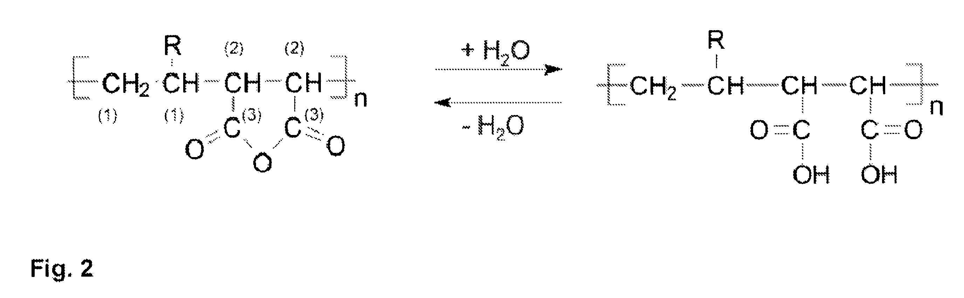

Für die Präparation der Lösungen zur Beschichtung der Wells wurden die hydrolysierten Formen der Maleinsäureanhydrid-Copolymere genutzt. Dabei zeigt die Fig. 2 die Hydrolyse und die Wiedergewinnung der Anhydridfunktionen an Maleinsäureanhydrid-Copolymeren in Form einer Gleichung für eine umkehrbare Reaktion. Durch die Hydrolyse mit jeweils einem Wassermolekül wird eine cyclische Anhydridgruppe in zwei benachbarte Carbonsäuregruppen umgewandelt. Dementsprechend erfolgt bei der Kondensation die Entfernung eines Wassermoleküls von den benachbarten Carbonsäuregruppen und eine Recyclisierung zur Anhydridfunktion. Es können unterschiedliche Comonomere eingesetzt werden, die sich in der verwendeten Seitenkette R voneinander unterscheiden. Als Copolymere wurden Poly(ethylen-alt-maleinsäureanhydrid) (PEMA), Poly(propen-alt-maleinsäureanhydrid) (PPMA) und Poly(styren-alt-maleinsäureanhydrid) (PSMA) eingesetzt. Die Seitenketten R entsprechen bei PEMA WasserstoffAtomen (H) und bei PPMA Methylgruppen (-CH₃). Bei PSMA sind die Seitenketten Styren-Ringe. PSMA besitzt eine Molmasse von M_{PSMA} = 20.000 g* mol⁻¹ (Leuna-Werke AG, Deutschland) und wurde in Ethanol p.a. (VWR International, Deutschland) zu einer Konzentration von 0,1 % gelöst. PPMA mit einer Molmasse M_{PPMA} = 39.000 g* mol⁻¹ (Leuna-Werke AG, Deutschland) und PEMA mit einer Molmasse von M_{PEMA} = 125.000 g* mol⁻¹ (Aldrich, München, Deutschland) wurden in entionisiertem Wasser zu einer Konzentration von 0,1 % gelöst. Eine einzelnes Well wurde jeweils mit 50 µl einer Lösung gefüllt und die Lösung wurde darin getrocknet.

Somit wurden nach der Entstehung der Aminogruppen auf den Polystyren(PS)-Oberflächen der 96-Well-Platten Lösungen des hydrolysierten Poly(ethylen-alt-Maleinsäureanhydrid) beziehungsweise des Poly(propylen-alt-Maleinsäureanhydrid) in entionisiertem Wasser auf die 96-Well-Platten aufgebracht und darin eingetrocknet. Für hydrolysiertes Poly(styren-alt-maleinsäureanhydrid) (PSMA) wurden, wie bereits erwähnt, Ethanollösungen angewendet. Die Maleinsäureanhydrid-Copolymere wurden vorher hydrolysiert, wobei das Anhydrid jeweils in die Carbonsäureform überführt wurde, um sie jeweils löslich in Wasser oder Ethanol zu machen, weil unpolare Lösungsmittel, wie Methylethylketon oder Tetrahydrofuran, die Polystyren(PS)-Oberfläche lösen und beschädigen würden.

Die kovalente Bindung des Copolymers an die Aminogruppen wurde durch 48-stündiges Erhitzen der Platten bei 90°C erreicht. Ein anschließendes 24-stündiges Spülen in entionisiertem Wasser wurde für wasserlösliche Copolymere (PPMA, PEMA) angewandt, um ungebundenes Copolymer zu entfernen. Poly(styren-alt-maleinsäureanhydrid) wurde in wässrigem Phospatpuffer pH 7.4 (Sigma-Aldrich, Deutschland) durchgeführt, um die bessere Löslichkeit des hydrolysierten Copolymers bei pH 7.4 zu nutzen. Um eine Salzbildung vor der Anhydridrückbildung zu unterdrücken, wurden diese Proben (PSMA) zuvor in 0.01 n Salzsäure (Applichem, Deutschland) und anschließend in entionisiertem Wasser gespült. Die Anhydridfunktionen für Biomolekülkopplungen wurden gemäß der Rückreaktion in Fig. 2 durch 48-stündiges Erhitzen bei 90 °C reaktiviert.

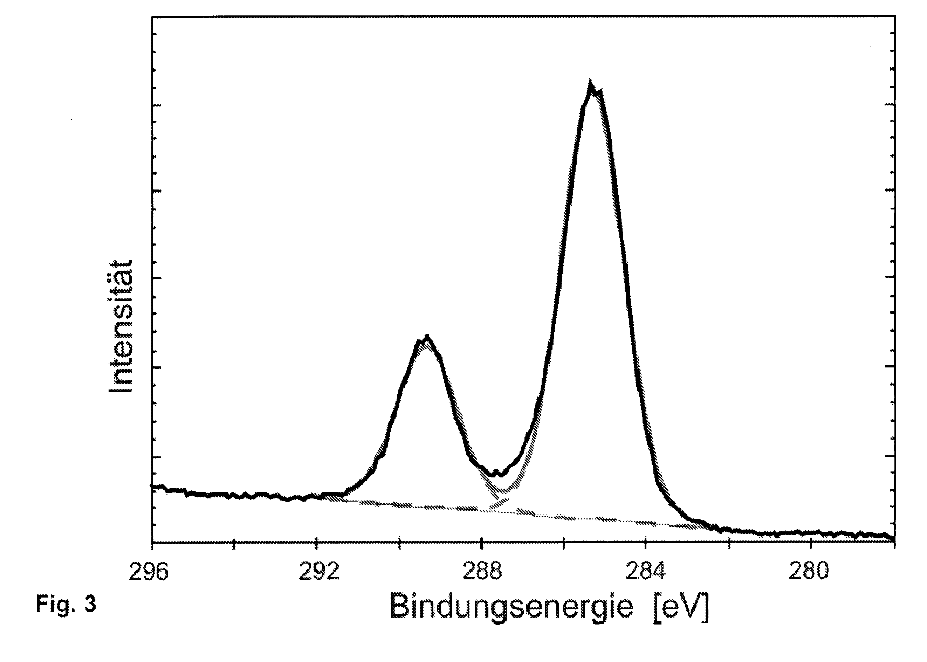

Das hoch aufgelöste Kohlenstoff-C₁ₛ-Spektrum in **Fig. 3** zeigt beispielhaft für eine Beschichtung mit Poly(ethylen-alt-Maleinsäureanhydrid) PEMA neben dem Hauptpeak bei 285,3 eV, der den Kohlenstoffatomen des Copolymers an den Positionen (1) und (2) gemäß **Fig. 2** zuzuordnen ist, einen weiteren Peak für Sauerstoff gebundenen Kohlenstoff im Anhydridring bei 289,4 eV, was der Position (3) in **Fig. 2** entspricht. Das Verhältnis von beiden Peaks [C_{289 eV}]: [C_{285 eV}] bietet eine weitere Indikation für die Schichtdicke, da über 10 nm Schichtdicke meist keine Kohlenstoffsignale aus dem Polystyren-Substrat mehr gemessen werden sollten. Die theoretischen Verhältnisse von beiden Peaks [C_{289 eV}]: [C_{285 eV}] betragen für Poly(ethylen-alt-Maleinsäureanhydrid) PEMA 0,5, für Poly(propen-alt-maleinsäureanhydrid) PPMA 0,4 und für eine reine Polystyren(PS)-Oberfläche 0. Obwohl der theoretische Wert nicht voll erreicht wird, demonstrieren die hohen Werte in **Tabelle 1** eine Schichtdicke im Bereich von 10 nm.

Um die Dicke des Copolymerfilms zu ermitteln, wurden der Stickstoffanteil und das hoch aufgelöste Kohlenstoff-C₁ₛ-Spektrum, wie in **Tabelle 1** gezeigt, quantifiziert. Das Stickstoffsignal aus der Aminfunktionalisierung der Polystyren(PS)- Oberfläche kann durch die Abdeckung durch die Copolymerphase als exponentiell abgeschwächt erwartet werden, was auf die begrenzte mittlere freie Weglänge der emittierten Photoelektronen zurückzuführen ist. **Tabelle 1** zeigt die Ergebnisse der Röntgenphotoelektronenspektroskopie (XPS)-Quantifizierung von PEMA- und PPMA- -Schichten auf Amin-funktionalisierten Polystyrenoberflächen von typischen Proben aus vier unabhängigen Experimenten. Der Stickstoffgehalt und das Verhältnis der C₁ₛ-Peaks bei 289,4 eV und 285,3 eV wird einmal vor und nach dem Abspülen in entionisiertem Wasser gemessen.

Um die nicht kovalent angebundenen Copolymere zu entfernen, wurden die PEMA- und PPMA- beschichteten Oberflächen mit entionisiertem Wasser 24 Stunden lang gespült. Für PSMA wurde dieser Spülschritt in Phosphatpuffer pH 7.4 durchgeführt. Aufgrund der Hydrolyse in der wässrigen Umgebung erhalten die Copolymere PEMA , PPMA und PSMA ihre Löslichkeit in wässriger Umgebung zurück und das ungebundene Copolymer wird von der Oberfläche abgelöst. Die XPS-Quantifizierung gemäß **Tabelle 1** sowie die Ellipsometriemessungen von Modellproben gemäß **Tabelle 2** beweisen den erfolgreichen Abspülungsschritt. Die verbleibende Copolymerschicht wird aus den XPS-Messungen geschätzt auf 8,4 nm für PEMA und 3,7 nm für PPMA. Die Ellipsometriemessungen (7,8 nm für PEMA und 4,2 nm für PPMA) bestätigen diese Werte und zeigen auch für PSMA eine monomolekulare Copolymerschicht von 4,4 nm Dicke. Die verschiedenen Schichtdicken sind durch die unterschiedlichen Molekulargewichte von 125.000 g*mol⁻¹ (PEMA), 39.000 g*mol⁻¹ (PPMA) sowie 20.000 g*mol⁻¹ (PSMA) und die unterschiedlichen sterischen Eigenschaften der Seitenketten zu erklären, was zu einer unterschiedlichen Materialbindung an der Polystyren-Oberfläche während der Ablagerung aus der gelösten Phase führt.

Die laterale Mikrostrukturierung der funktionalisierten Oberfläche wurde durch Vernetzen einer Polyoxyethylene-polyoxypropylene-Block-Copolymer mittels eines Niederdruck-Argonplasma erreicht. Dazu wurde das Polyoxyethylene-polyoxypropylene-Block-Copolymer (Pluronic F-68, BASF, Deutschland) 1 Stunde aus einer wässrigen Lösung adsorbiert. In dieser Zeit adsorbiert eine 3,8 nm dicke Schicht, welche ellipsometrisch auf Modelloberflächen nachgewiesen wurde. Während des Niederdruck-Argonplasma mit einer Leistung von 100 W, einem Argongasfluss von 40 Standard-cm³ pro Minute und einem Druck von 5 * 10⁻³ mbar wurden runde Siliziummasken mit einem Durchmesser von 5,5 mm (GeSiM, Großerkmannsdorf, Deutschland) mit kreisförmigen und streifenförmigen Bereichen in der Größe von 25 µm bis 80 µm angewendet. Die Behandlungszeit für das Plasmavernetzen wurde im Bereich von 3 s bis zu 10 s variiert, um die optimale Behandlungszeit zu bestimmen. Auf der Basis des Ergebnisses von entsprechenden Optimierungsversuchen wurde eine Zeit von 7 s als Behandlungszeit für die Niederdruck-Argonplasma-Behandlung gewählt. In dem Vernetzungsschritt wurde mittels des Niederdruck-Argonplasmas die Polyoxyethylene-polyoxypropylene-Block-Copolymerschicht lokal angebunden. Durch Spülen in entionisiertem Wasser für 24 Stunden wurde ungebundenes Copolymer anschließend beseitigt. Die verbleibende Polyoxyethylene-polyoxypropylene-Block-Copolymerschicht hat eine ellipsometrisch bestimmte Dicke von 1,1 nm und vermindert entscheidend die Proteinadsorption, wie es in Tabelle 3 anhand der mikroskopischen Helligkeitsmessung von fluoreszentmarkiertem Rinderserumalbumin zu sehen ist. Nur in den Bereichen ohne Polyoxyethylene-polyoxypropylene-Block-Copolymerschicht erfolgt eine Anlagerung von Protein, so dass mikrometergroße Bereiche entstehen, welche mit Protein belegt sind. Wird anstelle Albumin beispielsweise das Protein Fibronektin an diese Bereich angekoppelt, so können adhärent wachsende Zellen, wie zum Beispiel Endothelzellen lokale, in diesen Strukturen anwachsen.

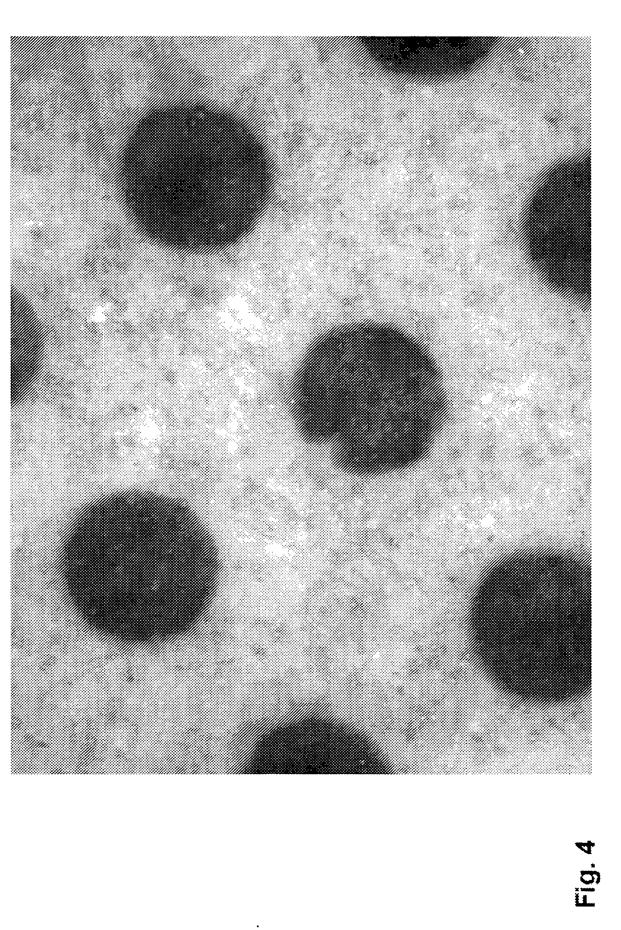

Die **Fig. 4** zeigt eine Oberfläche nach einer Mikrostrukturierung mit nachfolgender Anbindung von fluoreszenzmarkiertem Rinderserumalbumin. In den lokal beschichteten Bereichen kann nachfolgend kein Protein, das heißt in diesem Fall fluoreszenzmarkiertes Rinderserumalbumin, angebunden werden. Die beschichteten Löcher, an denen eine lokale Proteinanbindung unterbunden wird und die in Fig. 4 als Kreisflächen erscheinen, haben einen Durchmesser von 80 µm. Die Flächen erscheinen bei fluoreszenzmikroskopischer Betrachtung dunkel.

### LISTE DER BEZUGSZEICHEN UND ABKÜRZUNGEN

- 1: Position des Kohlenstoffs des Maleinsäureanhydrid-Copolymers
- 2: Position des Kohlenstoffs des Maleinsäureanhydrid-Copolymers
- 3: Position des Kohlenstoffs des Maleinsäureanhydrid-Copolymers
- PEMA: Poly(ethylen-alt-Maleinsäureanhydrid)
- PPMA: Poly(propen-alt-maleinsäureanhydrid)
- PSMA: Poly(styren-alt-maleinsäureanhydrid)
- P(x)MA: Allgemeine Abkürzung für das Maleinsäureanhydrid-Copolymer
- PEO: Polyoxyethylene-polyoxypropylene-Block-Copolymer

**Tabelle 1:**

| | **PEMA** | | **PPMA** | |
|---|---|---|---|---|
| | vor dem Abspülen | nach dem Abspülen | vor dem Abspülen | nach dem Abspülen |
| N[at.%] | 0,34 | 1,10 | 0,63 | 3.6 |
| [C_{289eV}]:[C_{285 eV}] | 0,38 | 0,37 | 0,32 | 0,18 |
| Schichtdicke [nm] | 13,1 | 8,4 | 10,6 | 3,7 |

**Tabelle 2:**

| Schichtdicke [nm] | **PEMA** | **PPMA** | **PSMA** |
|---|---|---|---|
| vor Spülen | 23,3 | 17,0 | 18,9 |
| nach Spülen | 7,8 | 4,2 | 4,4 |

**Tabelle 3:**

| Helligkeit [%] | **PEMA** | **PEO** |
|---|---|---|
| Albumin | 100 | 15% |

## Patentansprüche

1. Verfahren zur Funktionalisierung von Multi-Well-Platten aus Polystyren oder aus Polypropylen für biochemische Analysen und Zellkulturexperimente,
das die folgenden Verfahrenschritte umfasst:
a) Behandlung der Multi-Well-Platte mittels eines Ammoniak-Niederdruckplasmas, so dass reaktive Aminogruppen an der Plattenoberfläche entstehen;
b) Aufbringen einer wässrigen oder alkoholischen Lösung eines Maleinsäureanhydrid-Copolymers;
c) Eintrocknen der Lösung des Maleinsäureanhydrid-Copolymers auf der Oberfläche;
d) Wärmebehandlung zur kovalenten Bindung des Maleinsäureanhydrid-Copolymers an die Multi-Well-Platte;
e) Spülen mit wässriger Lösung zur Beseitigung von ungebundenem und wasserlöslichem Maleinsäureanhydrid-Copolymer und
f) Wärmebehandlung zur Wiederherstellung der Reaktivität der Anhydrid-Gruppen am Maleinsäureanhydrid-Copolymer.

2. Modifizierte Multi-Well-Platte nach Anspruch 1, **dadurch gekennzeichnet, dass** als Maleinsäureanhydrid-Copolymere Poly(styren-alt-maleinsäureanhydrid) POMA, Poly(propen-alt-maleinsäureanhydrid) PPMA und Poly(ethylen-alt-maleinsäureanhydrid) PEMA eingesetzt werden.

3. Modifizierte Multi-Well-Platte nach Anspruch 1 oder 2, **dadurch gekennzeichnet, dass** an die Anhydridgruppen bioaktive Moleküle kovalent gebunden werden.

4. Modifizierte Multi-Well-Platte nach Anspruch 1 oder 2, **dadurch gekennzeichnet, dass** an die Polymeroberflächen mit hydrolysierten Säuregruppen bioaktive Moleküle adsorptiv gebunden werden.

5. Modifizierte Multi-Well-Platte nach Anspruch 1 oder 2, d**adurch gekennzeichnet, dass** an die Anhydridgruppen Peptide über freie Aminogruppen kovalent gebunden werden.

6. Modifizierte Multi-Well-Platte nach Anspruch 3, **dadurch gekennzeichnet, dass** an die Anhydridgruppen Proteine über freie Aminogruppen kovalent gebunden werden.

7. Modifizierte Multi-Well-Platte nach Anspruch 4, **dadurch gekennzeichnet, dass** an die Polymeroberflächen mit hydrolysierten Säuregruppen Proteine adsorptiv gebunden werden.

8. Modifizierte Multi-Well-Platte nach Anspruch 3, **dadurch gekennzeichnet, dass** an die Anhydridgruppen Polysaccharide über freie OH-Gruppen kovalent gebunden werden.

9. Modifizierte Multi-Well-Platte nach Anspruch 1 oder 2, **dadurch gekennzeichnet, dass** der Funktionalisierung eine Mikrostrukturierung der Multi-Well-Platte folgt, indem eine Polyoxyethylene-polyoxypropylene-Block-Copolymerschicht mittels eines Niederdruck-Argonplasmas in einem Vernetzungsschritt lokal angebunden wird.

10. Modifizierte Multi-Well-Platte nach Anspruch 9, **dadurch gekennzeichnet, dass** als Maske Template aus Siliziumwafern dienen, welche vorgefertigte Öffnungen aufweisen, durch die das Argon-Niederdruckplasma hindurchdringen kann.

11. Modifizierte Multi-Well-Platte nach Anspruch 9 oder 10, **dadurch gekennzeichnet, dass** lateral chemisch heterogene Strukturen auf den Oberflächen der Multi-Well-Platte in einem Größenbereich von 5 µm bis 500 µm erreicht werden.

12. Modifizierte Multi-Well-Platte nach einem der Ansprüche 9 bis 11, **dadurch gekennzeichnet, dass** auf den Flächenbereichen des belassenen Maleinsäureanhydrid-Copolymers an die Anhydridgruppen bioaktive Moleküle kovalent gebunden werden.

13. Modifizierte Multi-Well-Platte nach Anspruch 12, **dadurch gekennzeichnet, dass** auf den Flächenbereichen des belassenen Maleinsäureanhydrid-Copolymers an die Anhydridgruppen Peptide über freie Aminogruppen kovalent gebunden werden.

14. Modifizierte Multi-Well-Platte nach Anspruch 12, **dadurch gekennzeichnet, dass** auf den Flächenbereichen des belassenen Maleinsäureanhydrid-Copolymers an die Anhydridgruppen Proteine über freie Aminogruppen kovalent gebunden werden.

15. Modifizierte Multi-Well-Platte nach Anspruch 12, **dadurch gekennzeichnet, dass** auf den Flächenbereichen des belassenen Maleinsäureanhydrid-Copolymers an die Anhydridgruppen Polysaccharide über freie OH-Gruppen kovalent gebunden werden.

16. Modifizierte Multi-Well-Platte nach einem der Ansprüche 9 bis 11, **dadurch gekennzeichnet, dass** auf den Flächenbereichen des belassenen Maleinsäureanhydrid-Copolymers an die Polymeroberflächen mit hydrolysierten Säuregruppen bioaktive Moleküle adsorptiv gebunden werden.

## Claims

1. A method for functionalisation of multi-well plates made of polystyrene or polypropylene for biochemical analyses and cell culture experiments, which method comprises the following method steps:
a) treatment of the multi-well plate by means of an ammonia low-pressure plasma, so that reactive amino groups are formed on the plate surface;
b) application of an aqueous or alcohol solution of a maleic anhydride copolymer;
c) drying of the solution of maleic anhydride copolymer on the surface;
d) heat treatment for covalent bonding of the maleic anhydride copolymer to the multi-well plate;
e) rinsing with aqueous solution to eliminate unbound and water-soluble maleic anhydride copolymer; and
f) heat treatment to re-establish the reactivity of the anhydride groups on the maleic anhydride copolymer.

2. The modified multi-well plate according to claim 1, **characterised in that** poly(styrene-alt-maleic anhydride) PSMA, poly(propene-alt-maleic anhydride) PPMA and poly(ethylene-alt-maleic anhydride) PEMA are used as maleic anhydride copolymers.

3. The modified multi-well plate according to either claim 1 or claim 2, **characterised in that** bioactive molecules are covalently bonded to the anhydride groups.

4. The modified multi-well plate according to either claim 1 or claim 2, **characterised in that** bioactive molecules are bonded adsorptively to the polymer surfaces with hydrolysed acid groups.

5. The modified multi-well plate according to either claim 1 or claim 2, **characterised in that** peptides are covalently bonded to the anhydride groups via free amino groups.

6. The modified multi-well plate according to claim 3, **characterised in that** proteins are covalently bonded to the anhydride groups via free amino groups.

7. The modified multi-well plate according to claim 4, **characterised in that** proteins are bonded adsorptively to the polymer surfaces with hydrolysed acid groups.

8. The modified multi-well plate according to claim 3, **characterised in that** polysaccharides are covalently bonded to the anhydride groups via free OH groups.

9. The modified multi-well plate according to either claim 1 or claim 2, **characterised in that** the functionalisation is followed by a microstructuring of the multi-well plate by locally bonding a polyoxyethylene-polyoxypropylene block copolymer layer in a cross-linking step by means of a low-pressure argon plasma

10. The modified multi-well plate according to claim 9, **characterised in that** templates formed of silicon wafers are used as a mask and have prefabricated openings through which the low-pressure argon plasma can penetrate.

11. The modified multi-well plate according to either claim 9 or claim 10, **characterised in that** laterally chemically heterogeneous structures are obtained on the surfaces of the multi-well plate in a size range of 5 µm to 500 µm.

12. The modified multi-well plate according to any one of claims 9 to 11, **characterised in that** bioactive molecules are covalently bonded to the anhydride groups on the surface regions of the maleic anhydride copolymer left.

13. The modified multi-well plate according to claim 12, **characterised in that** peptides are covalently bonded to the anhydride groups via free amino groups on the surface regions of the maleic anhydride copolymer left.

14. The modified multi-well plate according to claim 12, **characterised in that** proteins are covalently bonded to the anhydride groups via free amino groups on the surface regions of the maleic anhydride copolymer left.

15. The modified multi-well plate according to claim 12, **characterised in that** polysaccharides are covalently bonded to the anhydride groups via free hydroxyl groups on the surface regions of the maleic anhydride copolymer left.

16. The modified multi-well plate according to any one of claims 9 to 11, **characterised in that** bioactive molecules are adsorptively bonded to the polymer surfaces with hydrolysed acid groups on the surface regions of the maleic anhydride copolymer left.

## Revendications

1. Procédé de fonctionnalisation de plaques multi-puits en polystyrène ou en polypropylène destinées à des analyses biochimiques et des expériences de culture cellulaire, comprenant les étapes de procédé suivantes :
a) traitement de ladite plaque multi-puits au moyen d'un plasma d'ammoniac à basse pression, conduisant à la formation de groupes amino à la surface de ladite plaque ;
b) application d'une solution aqueuse ou alcoolique d'un copolymère d'anhydride maléique ;
c) séchage de la solution dudit copolymère d'anhydride maléique sur ladite surface ;
d) traitement thermique conduisant à la formation d'une liaison covalente entre ledit copolymère d'anhydride maléique et ladite plaque multi-puits ;
e) rinçage avec une solution aqueuse, afin d'enlever le copolymère d'anhydride maléique qui est resté non-lié et hydrosoluble, et
f) traitement thermique afin de rétablir la réactivité der groupes anhydride présents sur le copolymère d'anhydride maléique.

2. Plaque multi-puits modifiée selon la revendication 1, **caractérisée en ce qu'**en tant que copolymères d'anhydride maléique, on met en oeuvre du poly(styrène-alt-anhydride maléique) POMA, du poly(propène-alt-anhydride maléique) PPMA et du poly(éthylène-alt-anhydride maléique) PEMA.

3. Plaque multi-puits modifiée selon la revendication 1 ou 2, **caractérisée en ce que** l'on établit une liaison covalente entre lesdits groupes anhydride et des molécules bioactives.

4. Plaque multi-puits modifiée selon la revendication 1 ou 2, **caractérisée en ce que** l'on établit une liaison par adsorption entre les surfaces du polymère pourvues de groupes acide hydrolysés et des molécules bioactives.

5. Plaque multi-puits modifiée selon la revendication 1 ou 2, **caractérisée en ce que** l'on établit une liaison covalente entre lesdits groupes anhydride et des peptides, au travers de groupes amino libres.

6. Plaque multi-puits modifiée selon la revendication 3, **caractérisée en ce que** l'on établit une liaison covalente entre lesdits groupes anhydride et des protéines, au travers de groupes amino libres.

7. Plaque multi-puits modifiée selon la revendication 4, **caractérisée en ce que** l'on établit une liaison par adsorption entre la surface du polymère pourvue de groupes acide hydrolysés et des protéines.

8. Plaque multi-puits modifiée selon la revendication 3, **caractérisée en ce que** l'on établit une liaison covalente entre lesdits groupes anhydride et des polysaccharides, au travers de groupes OH libres.

9. Plaque multi-puits modifiée selon la revendication 1 ou 2, **caractérisée en ce que** ladite fonctionnalisation de la plaque multi-puits est suivie, dans une étape de réticulation, d'une microstructuration réalisée en établissant de façon localisée et au moyen d'un plasma d'argon à basse pression, une liaison avec une couche d'un copolymère à blocs de poly(oxyéthylène)-poly(oxypropylène).

10. Plaque multi-puits modifiée selon la revendication 9, **caractérisée en ce que** des gabarits sous forme wafers de silicium pourvus de ouvertures préfabriquées permettant le passage dudit plasma d'argon à basse pression, servent de masque.

11. Plaque multi-puits modifiée selon la revendication 9 ou 10, **caractérisée en ce que** l'on obtient, sur les surfaces de ladite plaque multi-puits, des structures à caractère chimique hétérogène dont les dimensions sont comprises entre 5 µm et 500 µm.

12. Plaque multi-puits modifiée selon l'une des revendication 9 à 11, **caractérisée en ce que** l'on établit, sur les parties de la surface dudit copolymère d'anhydride maléique laissées tel quel, une liaison covalente entre lesdits groupes anhydride et des molécules bioactives.

13. Plaque multi-puits modifiée selon la revendication 12, **caractérisée en ce que** l'on établit, sur les parties de la surface dudit copolymère d'anhydride maléique laissées tel quel, une liaison covalente entre lesdits groupes anhydride et des peptides, au travers de groupes amino libres.

14. Plaque multi-puits modifiée selon la revendication 12, **caractérisée en ce que** l'on établit, sur les parties de la surface dudit copolymère d'anhydride maléique laissées tel quel, une liaison covalente entre lesdits groupes anhydride et des protéines, au travers de groupes amino libres.

15. Plaque multi-puits modifiée selon la revendication 12, **caractérisée en ce que** l'on établit, sur les parties de la surface dudit copolymère d'anhydride maléique laissées tel quel, une liaison covalente entre lesdits groupes anhydride et des polysaccharides, au travers de groupes OH libres.

16. plaque multi-puits modifiée selon l'une des revendications 9 à 11, **caractérisée en ce que** l'on établit, sur les parties de la surface dudit copolymère d'anhydride maléique laissées tel quel, une liaison par adsorption entre les surfaces du polymère pourvues de groupes acide hydrolysés et des molécules bioactives.
